# NEUE EUROPÄISCHE PATENTSCHRIFT

(11) **EP 0 215 800 B2**
(45) Veröffentlichungstag und Bekanntmachung des Hinweises auf die Entscheidung über den Einspruch: **04.05.1994**
(45) Hinweis auf die Patenterteilung: 04.04.1990
(21) Anmeldenummer: 86900144.6
(22) Anmeldetag: 20.12.1985
(51) Int. Cl.: C07D 239/26, C07D 239/30, C09K 19/34

(54) **PYRIMIDINE**
PYRIMIDINES
PYRIMIDINES

(30) Priorität: 12.01.1985 DE 3500909
(43) Veröffentlichungstag der Anmeldung: 01.04.1987
(73) Patentinhaber: MERCK PATENT GmbH, D-64271 Darmstadt (DE)
(72) Erfinder: KRAUSE, Joachim, D-6110 Dieburg (DE); EIDENSCHINK, Rudolf, D-6115 Münster (DE); HITTICH, Reinhard, D-6101 Modautal 1 (DE); SCHEUBLE, Bernhard, D-6146 Alsbach (DE)
(86) Internationale Anmeldenummer: EP8500732
(87) Internationale Veröffentlichungsnummer: WO8604060

(56) Entgegenhaltungen:
- EP-A- 0 169 327
- EP-A- 0 191 860
- DD-A-09 589 2
- DE-A- 2 547 737
- DE-A- 3 333 168
- FR-A- 2 164 702
- Zaschke, Demus. Flüssige Kristalle in Tabellen, Band 2, 1984
- Zaschke, Stollte. Z. Chem. 15, 1975. Band 2 pp. 441-443
- Zaschke, Dissertation, Universität von Halle. 1977

## Beschreibung

Die Erfindung betrifft Verbindungen der Formel I 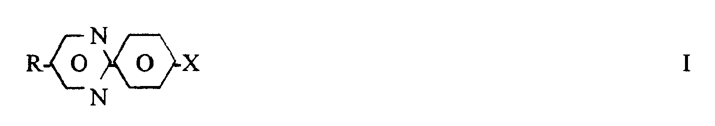
worin
- R: eine Alkylgruppe mit 1-15 C-Atomen, worin auch eine CH₂-Gruppe durch -O-ersetzt sein kann,
- X: O-CH₂-R¹, Y Halogen und
- R¹: eine Alkylgruppe mit 1-15 C-Atomen, worin eine oder mehrere nicht benachbarte CH₂-Gruppen durch -O- und/oder -CHY- ersetzt sind,

bedeutet, wobei für die Vertragsstaaten CH, DE, FR, GB und LI Pyrimidine der folgenden Formeln ausgenommen sind:

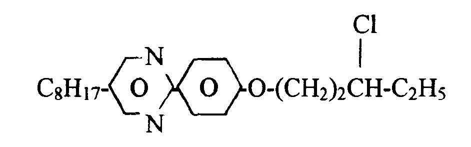

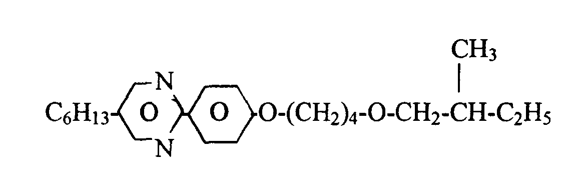

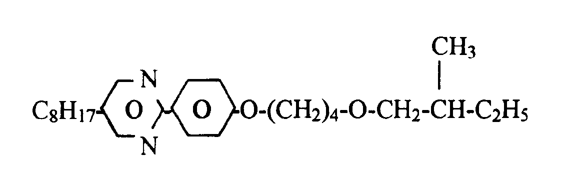

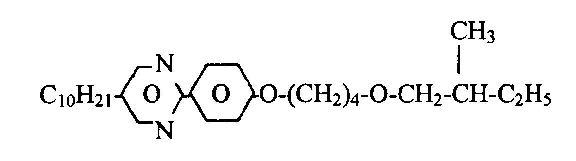

Diese Verbindungen sind Gegenstand der älteren, jedoch nicht vorpublizierten EP 0 191 860.

Der Einfachheit halber bedeuten im folgenden Phe eine 1,4-Phenylengruppe und Pyr eine Pyrimidin-2,5-diylgruppe.

In den Dokumenten EP-A-169 327, DE-A-25 47 737, FR-A-21 64 702, DD-95 892; H. Zaschke, Dissertation B, Universität Halle (Saale), 1977; Zaschke, Stolle, Z. Chem. 15 (1975), II, S. 441-443 und Zaschke, Demus, Flüssigkristalle in Tabellen, VEB Leipzig, Band II, 1984, werden ähnliche Verbindungen beschrieben.

Die Verbindungen der Formel I können wie ähnliche Verbindungen als Komponenten flüssigkristalliner Phasen verwendet werden, insbesondere für Displays, die auf dem Prinzip der verdrillten Zelle (TN-Displays), dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen für TN-Displays mit hohen Multiplextraten herstellbar.

Die zweikernigen Verbindungen der Formel I weisen besonders günstige elastische Konstanten und hervorragende Tieftemperaturstabilität auf. Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu variieren oder um die elastischen Eigenschaften zu verbessern. Die Verbindungen der Formel I eignen sich ferner als Zwischenprodukte zur Herstellung anderer Substanzen, die sich als Bestandteile flüssigkristalliner Dielektrika verwenden lassen. Teilweise zeigen die Verbindungen der Formel I lediglich monotrope flüssigkristalline Eigenschaften. Als Bestandteile flüssigkristalliner Dielektrika sind jedoch auch solche Verbindungen vorzüglich geeignet.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I. Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Vor- und nachstehend haben R, R¹, X und Y die angegebenen Bedeutung, sofern nicht ausdrücklich etwas anderes vermerkt ist.

In den Verbindungen der vor- und nachstehenden Formeln bedeutet R vorzugsweise Alkyl, ferner Alkoxy oder Oxaalkyl.

Die Alkylreste in den Gruppen R und/oder R¹ können geradkettig oder verzweigt sein. Vorzugsweise sind sie geradkettig, haben 2, 3, 4, 5, 6, 7, 8, 9, 10, 11 oder 12 C-Atome und bedeuten demnach bevorzugt Ethyl, Propyl, Butyl, Pentyl, Hexyl, Heptyl, Octyl, Nonyl, Decyl, Undecyl oder Dodecyl, ferner Methyl, Tridecyl, Tetradecyl oder Pentadecyl.

Falls diese Reste Alkylreste bedeuten, in denen eine ("Alkoxy" bzw. "Oxaalkyl") oder zwei ("Alkoxyalkoxy" bzw. "Dioxaalkyl") CH₂-Gruppen durch O-Atome ersetzt sind, so können sie geradkettig oder verzweigt sein. Vorzugsweise sind sie geradkettig, haben 2, 3, 4, 5, 6 oder 7 C-Atome und bedeuten demnach bevorzugt Ethoxy, Propoxy, Butoxy, Pentoxy, Hexoxy, Heptoxy, 2-Oxapropyl (= Methoxymethyl), 2-(=Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methoxy, Octoxy, Nonoxy, Decoxy, 2-, 3-, 4-, 5-, 6- oder 7-Oxaoctyl, 2-, 3-, 4-, 5-, 6-, 7- oder 8-Oxa-nonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formel I mit verzweigten Flügelgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste sind Isopropyl, 2-Butyl (= 1-Methyl-propyl), Isobutyl (= 2-Methylpropyl), 2-Methylbutyl, Isopentyl (= 3-Methylbutyl), 2-Methylpentyl, 3-Methyl-pentyl, 2-Ethylhexyl, 2-Propylpentyl, 2-Octyl, Isopropoxy, 2-Methylpropoxy, 2-Methylbutoxy, 3-Methylbutoxy, 2-Methylenpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy.

Unter den Verbindungen der Formel I sind diejenigen bevorzugt, in denen mindestens einer der darin enthaltenen Reste eine der angegebenen bevorzugten Bedeutungen hat.

Besonders bevorzugt sind Verbindungen der Formel I, worin R eine geradkettige Alkylgruppe mit 1 bis 10 C-Atomen.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der Fachmann kann durch Routinemethoden entsprechende Synthesemethoden aus dem Stand der Technik entnehmen.

Die Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

Verbindungen der Formel I können beispielsweise durch Umsetzung von in 2-Position entsprechend substituierten 3-Methoxyacrolein-Derivaten mit einem Säureadditionsalz eines entsprechenden Amidins in Gegenwart einer Base erhalten werden (DE-OS 31 40 868).

Die Umsetzung eines Acroleinderivates mit einem Säureadditionssalz eines Amidins wird zweckmäßig in Wasser oder einem organischen Lösungsmittel, beispielsweise einem Alkohol wie Methanol, Ethanol, Ethylenglykol und dergleichen, in Gegenwart einer Base durchgeführt. Bevorzugte Lösungsmittel sind Methanol und Ethanol. Die Additionssalze können Salze der Chlorwasserstoffsäure, Bromwasserstoffsäure, Schwefelsäure, p-Toluolsulfonsäure und dergleichen sein. Vorzugsweise wird jedoch das Hydrochlorid verwendet. Bevorzugte Basen sind die Alkalimetallalkoholate, insbesondere Natriummethylat und Natriumethylat. Temperatur und Druck sind keine kritischen Aspekte in dieser Reaktion. Zweckmäßigerweise werden Atmosphärendruck und eine Temperatur zwischen Raumtemperatur und Rückflußtemperatur, vorzugsweise Raumtemperatur, angewendet.

Verbindungen der Formel I können weiterhin durch Umsetzung entsprechend substituierter Malondialdehyde bzw. deren reaktionsfähigen Derivaten mit einem Säureadditionssalz eines entsprechenden Amidins erhalten werden.

Als reaktionsfähige Derivate der Malondialdehyde kommen in erster Linie Tetraalkylacetale in Betracht, worin Alkyl Niederalkyl mit 1 bis 5 Kohlenstoffatomen, vorzugsweise Methyl oder Ethyl, bedeutet. Die Umsetzung wird zweckmäßig in einem organischen Lösungsmittel, wie beispielsweise Dimethylsulfoxid, Dimethylformamid oder Hexamethylphosphorsäuretriamid, durchgeführt. Als Additionssalze der entsprechenden Amidine werden vorzugsweise die oben beschriebenen Salze eingesetzt.

Temperatur und Druck sind keine kritischen Aspekte in der Umsetzung. Zweckmäßigerweise werden Atmosphärendruck und Temperaturen zwischen Raumtemperatur und 200°, vorzugsweise 100° bis 180°, angewendet.

Verbindungen der Formel I, worin R¹ eine Alkylgruppe bedeutet, worin mindestens eine CH₂-Gruppe durch -O- ersetzt ist, sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfonat umgesetzt werden, zweckmäßig in einem inerten Lösungsmittel wie Aceton, DMF oder Dimethylsulfoxid oder auch einem Überschuß an wäßriger oder wäßrigalkoholischer NaOH oder KOH bei Temperaturen zwischen 20° und 100°.

Die erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder -cyclohexyl-ester, Phenylcyclohexane, Cyclohexylbiphenyl, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Bis-cyclohexylethane, 1,2-Bis-phenylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

R'-L-G-E-R'' II

worin L und Eje ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe, 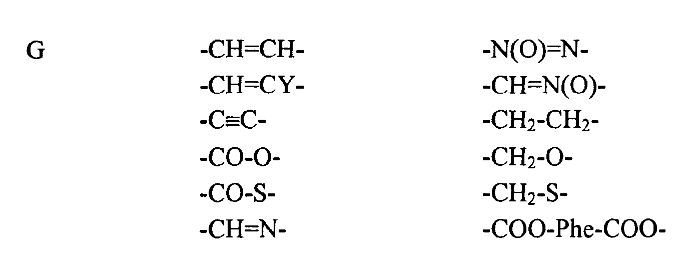
oder eine C-C-Einfachbindung, Y Halogen, vorzugsweise Chlor, oder -CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, NO₂, CF₃, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle dieser Substanzen sind nach literaturbekannten Methoden herstellbar.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße Dielektrika enthaltend 0,1 bis 40, vorzugsweise 0,5 bis 30 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.
Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecylammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. V or- und nachstehend bedeuten Prozentangaben Gewichtsprozent; alle Temperaturangaben sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

### Referenz-Beispiel

Ein Gemisch von 25,6 g 2-(4-Hydroxyphenyl)-5-n-hexylpyrimidin, 17,5 g Bromessigsäureethylester und 14,5 g Kaliumcarbonat wird in 75 ml Dimethylformamid 12 Std. unter Rühren auf 100° erhitzt. Man saugt vom Salz ab, engt zum Rückstand ein, nimmt in Ether auf und wäscht mit Wasser neutral. Die übliche Aufarbeitung ergibt 4-(5-n-Hexylpyrimidin-2-yl)-phenoxyessigsäureethylester.

### Beispiel 1

Analog Referenz-Beispiel erhält man aus 2-(4-Hydroxyphenyl)-5-n-octylpyrimidin und dem Methansulfonat des Ethylenglycolmonoethylethers 2-[4-(3-Oxapentyl)-oxy-phenyl]-5-n-octylpyrimidin.
2-[4-(3-Oxapentyl)-oxy-phenyl]-5-propylpyridimin
2-[4-(3-Oxapentyl)-oxy-phenyl]-5-butylpyridimin
2-[4-(3 -Oxapentyl)-oxy-phenyl]-5-pentylpyridimin
2-[4-(3-Oxapentyl)-oxy-phenyl]-5-hexylpyridimin
2-[4-(3 -Oxapentyl)-oxy-phenyl]-5-heptylpyridimin
2-[4-(3-Oxapentyl)-oxy-phenyl]-5-nonylpyridimin
2-[4-(3-Oxapentyl)-oxy-phenyl]-5-decylpyridimin
2-[4-(2-Oxapentyl)-oxy-phenyl]-5-propylpyridimin
2-[4-(2-Oxapentyl)-oxy-phenyl]-5-butylpyridimin
2-[4-(2-Oxapentyl)-oxy-phenyl]-5-pentylpyridimin
2-[4-(2-Oxapentyl)-oxy-phenyl]-5-hexylpyridimin
2-[4-(2-Oxapentyl)-oxy-phenyl]-5-heptylpyridimin
2-[4-(2-Oxapentyl)-oxy-phenyl]-5-nonylpyridimin
2-[4-(2-Oxapentyl)-oxy-phenyl]-5-decylpyridimin
2-[4-(3-Oxahexyl)-oxy-phenyl]-5-propylpyridimin
2-[4-(3-Oxahexyl)-oxy-phenyl]-5-butylpyridimin
2-[4-(3-Oxahexyl)-oxy-phenyl]-5-pentylpyridimin
2-[4-(3-Oxahexyl)-oxy-phenyl]-5-hexylpyridimin
2-[4-(3-Oxahexyl)-oxy-phenyl]-5-heptylpyridimin
2-[4-(3-Oxahexyl)-oxy-phenyl]-5-nonylpyridimin
2-[4-(3-Oxahexyl)-oxy-phenyl]-5-decylpyridimin
2-[4-(3-Oxabutyl)-oxy-phenyl]-5-propylpyridimin
2-[4-(3-Oxabutyl)-oxy-phenyl]-5-butylpyridimin
2-[4-(3-Oxabutyl)-oxy-phenyl]-5-pentylpyridimin
2-[4-(3-Oxabutyl)-oxy-phenyl]-5-hexylpyridimin
2-[4-(3-Oxabutyl)-oxy-phenyl]-5-heptylpyridimin
2-[4-(3-Oxabutyl)-oxy-phenyl]-5-nonylpyridimin
2-[4-(3-Oxabutyl)-oxy-phenyl]-5-decylpyridimin

### Beispiel 2

Ein Gemisch von 5,4 g 2-(4-Hydroxyphenyl)-5-n-heptylpyrimidin, 23 g 1,5-Dibrompentan und 3 g Kaliumcarbonat wird in 50 ml DMF 12 Std. unter Rühren auf 100° erhitzt. Man arbeitet wie üblich auf und erhält 2-[4-(5-Brompentyl)-oxyphenyl]-5-n-heptylpyrimidin.

Analog werden hergestellt:
2-[4-(5-Brompentyl)-oxyphenyl]-5-propylpyridimin
2-[4-(5-Brompentyl)-oxyphenyl]-5-butylpyridimin
2-[4-(5-Brompentyl)-oxyphenyl]-5-pentylpyridimin
2-[4-(5-Brompentyl)-oxyphenyl]-5-hexylpyridimin
2-[4-(5-Brompentyl)-oxyphenyl]-5-nonylpyridimin

## Patentansprüche (Patentansprüche für folgende(n) Vertragsstaat(en): CH, DE, FR, GB, LI)

1. Pyrimidine der Formel I 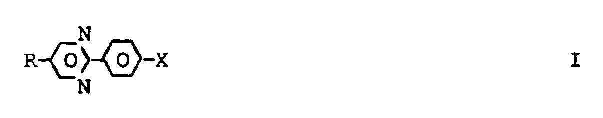 worin
R eine Alkylgruppe mit 1-15 C-Atomen, worin auch eine CH₂-Gruppe durch -O- ersetzt sein kann,
X -O-CH₂-R¹,
Y Halogen und
R¹ eine Alkylgruppe mit 1-15 C-Atomen, worin eine oder mehrere nicht benachbarte CH₂-Gruppen durch -O- und/oder -CHY- ersetzt sind,
bedeutet,
wobei Pyrimidine der folgenden Formeln ausgenommen sind:

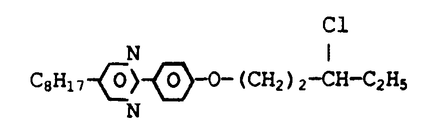

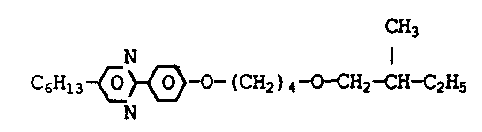

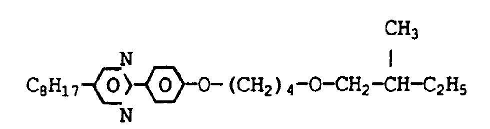

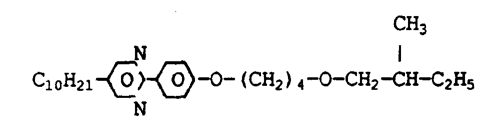

2. Pyrimidine nach Anspruch 1, dadurch gekennzeichnet, daß R¹ eine Alkylgruppe mit 1-15 C-Atomen bedeutet, worin eine CH₂-Gruppe durch -CHY- ersetzt ist, wobei Y Halogen bedeutet.

3. Pyrimidine nach einem der Ansprüche 1 und 2, dadurch gekennzeichnet, daß R eine Alkylgruppe mit 1-15 C-Atomen ist.

4. Pyrimidine nach Anspruch 3, dadurch gekennzeichnet, daß R eine geradkettige Alkylgruppe mit 2 bis 12 C-Atomen ist.

5. Verwendung der Verbindungen nach einem der Ansprüche 1 bis 4 als Komponenten flüssigkristalliner Phasen.

6. Flüssigkristalline Phase mit mindestens zwei flüssigkristallinen Komponenten, dadurch gekennzeichnet, daß mindestens eine Komponente eine Verbindung nach einem der Ansprüche 1 bis 4 ist.

7. Flüssigkristallanzeigeelement, dadurch gekennzeichnet, daß es eine Phase nach Anspruch 6 enthält.

8. Elektrooptisches Anzeigeelement nach Anspruch 7, dadurch gekennzeichnet, daß es als Dielektrikum eine Phase nach Anspruch 6 enthält.

## Patentansprüche (Patentansprüche für folgende(n) Vertragsstaat(en): AT, BE, IT, NL)

1. Pyrimidine der Formel I 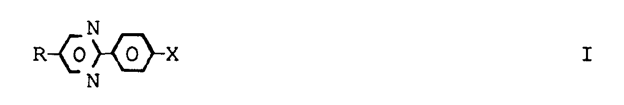 worin
R eine Alkylgruppe mit 1-15 C-Atomen, worin auch eine CH₂-Gruppe durch -O- ersetzt sein kann,
X -O-CH₂-R¹,
Y Halogen und
R¹ eine Alkylgruppe mit 1-15 C-Atomen, worin eine oder mehrere nicht benachbarte CH₂-Gruppen durch -O- und/oder -CHY-ersetzt sind,
bedeutet.

2. Pyrimidine nach Anspruch 1, dadurch gekennzeichnet, daß R¹ eine Alkylgruppe mit 1-15 C-Atomen bedeutet, worin eine CH₂-Gruppe durch -CHY- ersetzt ist, wobei Y Halogen bedeutet.

3. Pyrimidine nach einem der Ansprüche 1 und 2, dadurch gekennzeichnet, daß R eine Alkylgruppe mit 1-15 C-Atomen ist.

4. Pyrimidine nach Anspruch 3, dadurch gekennzeichnet, daß R eine geradkettige Alkylgruppe mit 2 bis 12 C-Atomen ist.

5. Verwendung der Verbindungen nach einem der Ansprüche 1 bis 4 als Komponenten flüssigkristalliner Phasen.

6. Flüssigkristalline Phase mit mindestens zwei flüssigkristallinen Komponenten, dadurch gekennzeichnet, daß mindestens eine Komponente eine Verbindung nach einem der Ansprüche 1 bis 4 ist.

7. Flüssigkristallanzeigeelement, dadurch gekennzeichnet, daß es eine Phase nach Anspruch 6 enthält.

8. Elektrooptisches Anzeigeelement nach Anspruch 7, dadurch gekennzeichnet, daß es als Dielektrikum eine Phase nach Anspruch 6 enthält.

## Claims (Claims for the following Contracting State(s): CH, DE, FR, GB, LI)

1. Pyrimidines of the formula I 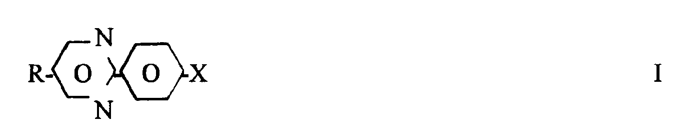 in which
R is an alkyl group of 1-15 C atoms, in which in addition one CH₂ group can be replaced by -O-,
X is -O-CH₂-R¹,
Y is halogen, and
R¹ is an alkyl group of 1-15 C atoms, in which one or more non-adjacent CH₂ groups are replaced by -O- and/or -CHY-,
whereby pyrimidines of the following formulae are disclaimed:

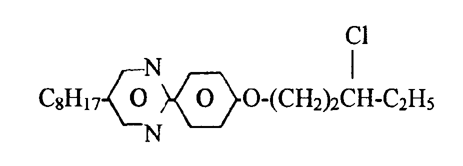

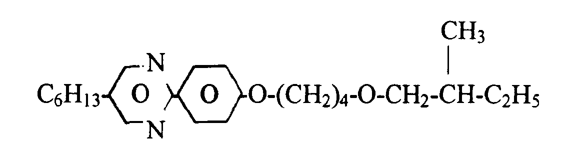

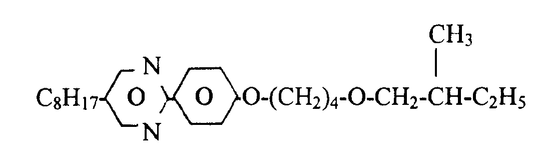

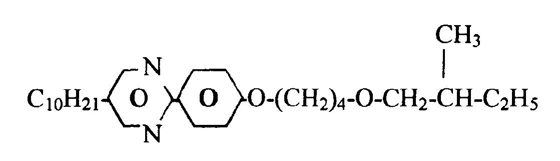

2. Pyrimidines according to Claim 1, characterized in that R¹ is an alkyl group of 1-15C atoms, in which one CH₂ group has been replaced by -CHY- where Y is halogen.

3. Pyrimidines according to one of claims 1 and 2, characterized in that R is an alkyl group of 1-15 C atoms.

4. Pyrimidines according to Claim 3, characterized in that R is a straight-chain alkyl group of 2 to 12 C atoms.

5. Use of the compounds according to one of the claims 1 to 4 as components of liquid-crystalline phases.

6. Liquid-crystalline phase having at least two liquid-crystalline components, characterized in that at least one component is a compound according to one of claims 1 to 4.

7. Liquid crystal display element, characterized in that it contains a phase according to claim 6.

8. Electro-optical display element according to claim 7, characterized in that it contains a phase according to claim 6.

## Claims (Claims for the following Contracting State(s): AT, BE, IT, NL)

1. Pyrimidines of the formula I 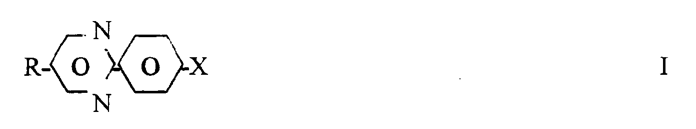 in which
R is an alkyl group of 1-15 C atoms, in which in addition one CH₂ group can be replaced by -O-,
X is -O-CH₂-R¹,
Y is halogen, and
R¹ is an alkyl group of 1-15 C atoms, in which one or more non-adjacent CH₂ groups are replaced by -O- and/or -CHY.

2. Pyrimidines according to Claim 1, characterized in that R¹ is an alkyl group of 1-15C atoms, in which one CH₂ group has been replaced by -CHY- where Y is halogen.

3. Pyrimidines according to one of claims 1 and 2, characterized in that R is an alkyl group of 1-15 C atoms.

4. Pyrimidines according to Claim 3, characterized in that R is a straight-chain alkyl group of 2 to 12 C atoms.

5. Use of the compounds according to one of the claims 1 to 4 as components of liquid-crystalline phases.

6. Liquid-crystalline phase having at least two liquid-crystalline components, characterized in that at least one component is a compound according to one of claims 1 to 4.

7. Liquid crystal display element, characterized in that it contains a phase according to claim 6.

8. Electro-optical display element according to claim 7, characterized in that it contains a phase according to claim 6.

## Revendications (Revendications pour l'(les) Etat(s) contractant(s) suivant(s): CH, DE, FR, GB, LI)

1. Pyrimidines de formule I 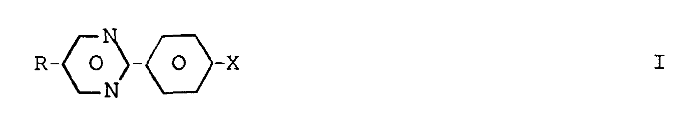 où
R représente un groupe alkyle contenant 1 à 15 atomes de C dans lequel un groupe CH₂ peut être remplacé par -O-,
X représente -O-CH₂-R¹,
Y représente un halogène et
R¹ représente un groupe alkyle contenant 1 à 15 atomes de C dans lequel un ou plusieurs groupes CH₂ non voisins sont remplacés par -O- et/ou -CHY-, avec la restriction que les composés suivants soient exclus:

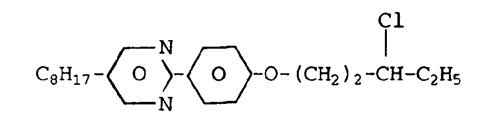

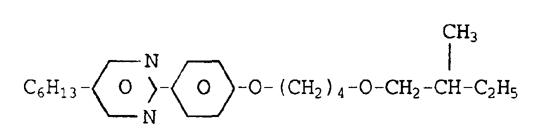

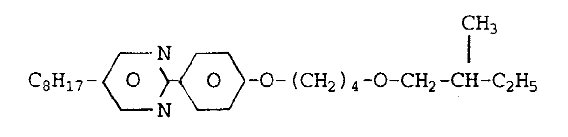

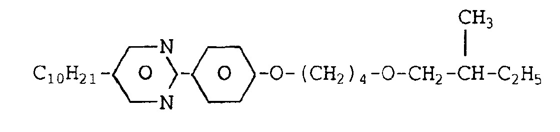

2. Pyrimidines selon la revendication 1, caractérisées par le fait que R¹ représente un groupe alkyle contenant 1 à 15 atomes de C ou un groupe CH₂ est remplacé par -CHY-, Y représentant un halogène.

3. Pyrimidines selon l'une des revendications 1 et 2, caractérisées par le fait que R représente un groupe alkyle contenant 1 à 15 atomes de C.

4. Pyrimidines selon la revendication 3, caractérisées par le fait que R représente un groupe alkyle linéaire contenant 2 à 12 atomes de C.

5. Utilisation des composés selon l'une des revendications 1 à 4 comme composants de phases à cristaux liquides.

6. Phase à cristaux liquides avec au moins deux composants à cristaux liquides, caractérisée par le fait qu'au moins un composant est un composé selon l'une des revendications 1 à 4.

7. Elément d'affichage à cristaux liquides, caractérisé par le fait qu'il contient une phase selon la revendication 6.

8. Elément d'affichage électro-optique selon la revendication 7, caractérisé par le fait qu'il contient une phase selon la revendication 6.

## Revendications (Revendications pour l'(les) Etat(s) contractant(s) suivant(s): AT, BE, IT, NL)

1. Pyrimidines de formule I 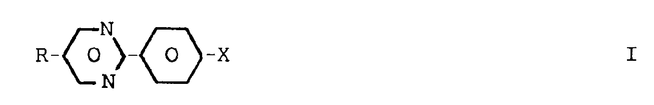 où
R représente un groupe alkyle contenant 1 à 15 atomes de C dans lequel un groupe CH₂ peut être remplacé par -O- et/ou -CHY-,
X représente -O-CH₂-R¹,
Y représente un halogène et
R¹ représente un groupe alkyle contenant 1 à 15 atomes de C dans lequel un ou plusieurs groupes CH₂ non voisins sont remplacés par -O- et/ou -CHY-.

2. Pyrimidines selon la revendication 1, caractérisées par le fait que R¹ représente un groupe alkyle contenant 1 à 15 atomes de C ou un groupe CH₂ est remplacé par -CHY-, Y représentant un halogène.

3. Pyrimidines selon l'une des revendications 1 et 2, caractérisées par le fait que R représente un groupe alkyle contenant 1 à 15 atomes de C.

4. Pyrimidines selon la revendication 3, caractérisées par le fait que R représente un groupe alkyle linéaire contenant 2 à 12 atomes de C.

5. Utilisation des composés selon l'une des revendications 1 à 4 comme composants de phases à cristaux liquides.

6. Phase à cristaux liquides avec au moins deux composants à cristaux liquides, caractérisée par le fait qu'au moins un composant est un composé selon l'une des revendications 1 à 4.

7. Elément d'affichage à cristaux liquides, caractérisé par le fait qu'il contient une phase selon la revendication 6.

8. Elément d'affichage électro-optique selon la revendication 7, caractérisé par le fait qu'il contient une phase selon la revendication 6.
